# EUROPEAN PATENT SPECIFICATION

(11) **EP 2 928 377 B1**
(45) Date of publication and mention of the grant of the patent: **13.03.2019**
(21) Application number: 13824190.6
(22) Date of filing: 10.12.2013
(51) Int. Cl.: A61F 5/00

(54) **ASSEMBLY OF AN IMMOBILISATION MASK, A CARRIER AND A CONNECTING PROFILE, AND USE THEREOF**
SYSTEM AUS IMMOBILIERUNGSMASKE, PATIENTENLIEGE UND VERBINDENDEM PROFIL, UND DESSEN BENUTZUNG
SYSTÈME D'UN MASQUE D'IMMOBILISATION, SUPPORT ET PROFILÉ À CONNECTION, ET SON UTILISATION

(30) Priority: 10.12.2012 BE 201200832
(43) Date of publication of application: 14.10.2015
(73) Proprietor: Orfit Industries, 2110 Wijnegem (BE)
(72) Inventor: DE GRUYTERE, Simon, 2450 Meerhout (BE)
(74) Representative: Gevers Patents
(86) International application number: PCT/IB2013/060779
(87) International publication number: WO 2014/091407

(56) References cited:
- EP-A1- 1 537 831
- EP-A1- 1 900 326
- EP-A1- 2 141 009
- WO-A1-2004/045481
- WO-A2-00/27331
- WO-A2-2004/047611
- DE-U1-202011 001 492
- US-A- 3 188 079
- US-A- 5 988 173
- US-A1- 2005 051 172
- US-A1- 2006 253 985
- US-A1- 2008 078 031
- US-B1- 6 244 270
- US-B1- 6 490 737
- US-B1- 6 698 045

## Description

This invention concerns an assembly of a carrier for supporting a patient and an immobilisation mask that is connectable to the carrier for immobilizing a body part of the patient on the carrier, the carrier and the immobilisation mask containing cooperating connection means, as described in the first claim.

The use of masks or similar means for positioning one or more body parts of a patient in a predetermined position on the carrier and immobilizing of the body part in this position in a predetermined posture during the treatment of the patient with radiation, such as for example with Intensity Modulated Radiation Therapy (IMRT), Image Guided Radiation Therapy (IGRT), stereotactic radiation therapy or surgery and proton therapy, is well known.

Such masks are described for example in US5988173, DE202011001492, US6244270, WO00/27331 and EP1900326.

With such techniques, the accurate and reproductive positioning of the body part to be treated of a predetermined position in a predetermined posture is of utmost importance. As use is often made of a radiation source that has the shape and/or size of the tissue to be treated, it is therefore essential that the radiation is directed at the tumour, and to spare the surrounding healthy tissue. Deviations of a few mm may result in that not the tumour is irradiated, but the surrounding healthy tissue, which is thus destroyed or damaged. This is undesirable.

Treatment and imaging techniques that make use of charged, heavy particles such as protons, offer the advantage in comparison to other radiation sources such as electrons, photons, gamma of X-radiation that the radiation dose can be better directed to the tissue to be treated. Proton beams usually scatter less in the tissue to be treated an exhibit a low lateral dispersion, which limits the risk of lateral damage. This offers the advantage that a directed killing of tumour cells can be obtained and that the surrounding healthy tissue can be spared. In order to enable the directed killing of unwanted tissue with both more or less dispersing radiation beams, an accurate positioning of the body part to be treated in relation to the radiation source is essential. To minimize the risk of unwanted irradiation of surrounding tissue, it is not only required that the body part is accurately positioned in the desired posture in relation to the radiation source during the treatment, but also that the body part is fixated during the treatment in the intended position in the intended posture and that the range of motion during the treatment is limited to a minimum. An accurate and reproducible positioning is of importance during fractionated treatment in which the patient is administered repeated radiation doses of less than optimal dose with in-between time intervals of typically a couple of days or weeks, to ensure sufficient radiation of the area to be treated and to limit the irradiation of healthy tissue to a minimum. An accurate positioning is also exceptionally important during a single treatment, in which a large radiation dose is administered once. Various tools have been designed to realize an accurate and reproducible positioning of a body part in relation to a radiation source, both with regard to the position as well as the posture, and thereby also to limit the range of motion of the body part to a minimum.

Traditionally, a patient is placed on a table, on which a base plate or carrier is located. Base plates that are used with radio therapy are often manufactured as a sandwich structure with a foam core and carbon fibre skin. Immobilizing a body part of a patient such as for example the head or any other body part, on a particular place on the table in a predetermined posture is realized by attaching an immobilisation mask to the base plate or the table. The immobilisation mask has a shape that closely matches the body part to be immobilized, to limit the range of motion of the body part to be immobilized.

EP1537831 in the name of Orfit discloses a non-invasive immobilisation mask for immobilizing for example the head of a patient in a predetermined position on a base plate or carrier, in a predetermined posture. The mask is manufactured out of a sheet of a thermoplastic material which is formed such that it matches the shape of the body part to be immobilized as closely as possible. To enable optimum design, a thermoplastic material is chosen with a low melting temperature, such that the material can be shaped directly onto the body part. An example of a suitable material is ε-polycaprolactone. Means are provided to at least a section of the edge of the mask for attaching the mask to the base plate. The attachment means disclosed in EP1537831 comprise a rail that extends along the edge or edges of the mask, the rail engages a corresponding slot in the base plate. The presence of the slot implies that the homogeneity of the material from which the base plate is made up, is interrupted, such that the homogeneity of the radiation beam that reaches the patient is adversely affected.

To meet the special requirements set by the radiation therapy concerning the immobilizing of the patient in relation to the radiation source and, especially with proton therapy, homogeneity of the radiation that reaches the tissue to be treated, a so-called base plate or carrier is designed that supports the patient well.

An example of a base plate or carrier, used in practice, is provided along the edge with a plurality of openings for receiving connection pins that fit into corresponding openings in the edge of the immobilization mask for attaching the mask onto the base plate. The pins are generally commercially available and know many applications in various fields of application. They are, for example, manufactured from polyoxymethylene or another plastic, they are relatively small and have the disadvantage that they exhibit a low mechanical strength and that they break often when establishing the connection. As a result, not only the number of times that the pins can be reused, is limited, but also there is the problem that the pins can loosen during the treatment, which would compromise the accurate positioning of the patient. In addition, the mask is subjected to considerable stress when it is connected to the carrier, which greatly hampers the application of the connection pins.

Consequently, there is a need for attachment means for attaching an immobilisation mask to a carrier or base plate for the support of the patient, that exhibits an improved mechanical strength.

The aim of this invention consists therefore in supplying an assembly of a carrier for supporting a patient and an immobilisation mask, connectable with the carrier, wherein means are provided for attaching the immobilisation mask to the carrier that exhibit an improved mechanical strength and a longer service life.

This is achieved according to the invention with an assembly that displays the mechanical characteristics of the body of the first claim.

To this end, the assembly of this invention is characterized in that connection means contain at least one profile that is provided on a first side to be connected to, or even for example connected to, the immobilisation mask, and on a second side opposite the first side contains first connection means for connecting the immobilisation mask to the carrier, wherein the first connection means are provided to engage, in the connected state, corresponding connection means along the outer edge of the carrier.

A profile that engages an outer edge of the carrier is simple to attach, also if a tensile force must be exerted onto the profile and/or the mask with which the profile is connected, to establish the connection. With a profile that engages the outer edge of the carrier, such a force is easier to exert then with the use of relatively small pins, which detach relatively easy when subjected to a large tensile force. To ensure that mainly the tissue to be treated is irradiated and the risk of undesired irradiation of surrounding tissue is kept to a minimum, it is of importance that the body part to be immobilized is fixated during the treatment at the intended place and in the intended posture and that the range of motion during the treatment is limited to a minimum. Therefore, it is of importance that the immobilisation mask closely fits the body part to be immobilized, which implies that the material of the mask, but also the profile, in the connected state with the carrier, are subjected to great tensile force and stress. The connection means or the profile of this invention enable, despite a high tensile force on the profile, to establish and maintain a connection.

The profile preferably engages in the thickness direction onto the carrier, this is usually the direction wherein the tensile force and tension onto the immobilisation mask and profile are the greatest. Since the profile engages in the thickness direction, optimal transfer of forces from the mask to the carrier is accomplished.

The profile preferably contains a first lower lip which is provided, in the connected state, to extend along a lower surface of the carrier, wherein the edge of the carrier is held between said upper and lower lip. In a further preferred embodiment, this lip is resiliently arranged to facilitate the establishment and severing of the connection.

To promote an accurate and reproducible positioning of the profile onto the carrier, on a lower surface of the carrier a guide is preferably provided for guiding the displacement of the lower lip to the position where the lip engages the carrier, at the connection of the immobilisation mask to the carrier.

The invention is illustrated in the accompanying drawings which display preferred embodiments and describe the profile, the immobilisation mask provided with a profile, and an assembly of a carrier, an immobilisation mask and profile for the connection of the immobilisation mask to the carrier or base plate or carrier on which the patient rests. The invention is further illustrated in the description of the figures of these preferred embodiments. In the following figure description, the same reference numbers refer to the same elements.
Figure 1 shows a view of the lower side of the connection means (profile) for connecting an immobilisation mask with the carrier for supporting a patient or a portion thereof.
Figure 2 shows a cross-section along the line II-II in Figure 1.
Figure 3 shows a cross-section along the line III-III in Figure 1.
Figure 4 shows a view to the upper side of a carrier.
Figure 5 shows a view to the lower side of a carrier.

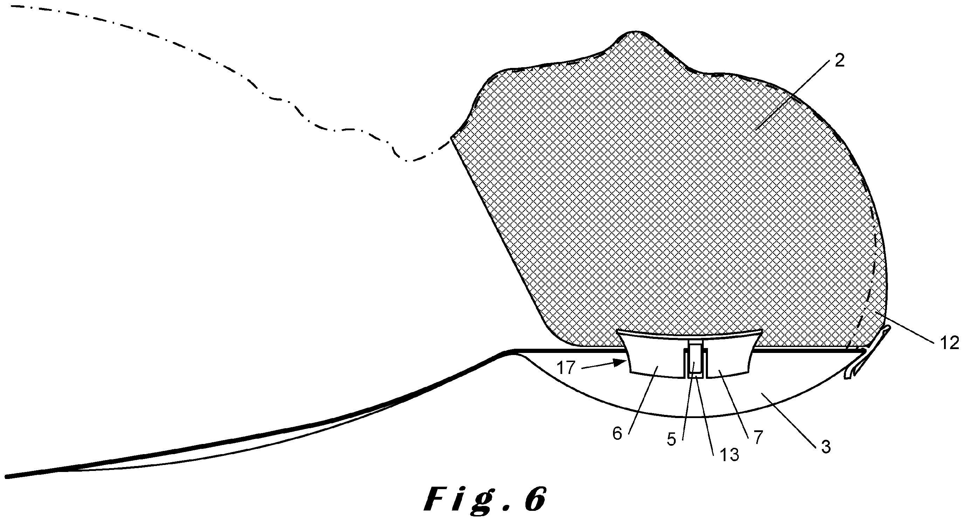

Figure 6 shows an immobilisation mask in the connected state with the carrier.
Figure 7a shows a view to the immobilisation mask connected with a carrier according to the state of the art. Figure 7b shows a cross-section of Fig. 7a. Figure 7c shows a view of the carrier used in Fig. 7a.

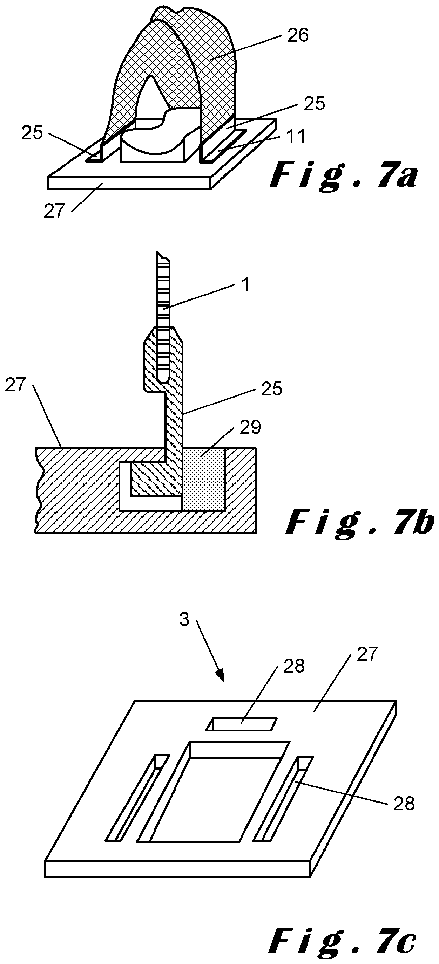

Figures 7a, 7b and 7c show the way in which in the state of the art established a connection between a mask 26 for immobilizing a body part of a patient, for example the head, onto a carrier 27, for example a carrier on which the patient rests, is established. As is shown in figure 7a it is known from the state of the art to provide an L-shaped profile 25 along the edge of the mask that engages an opening or slot 28, provided in the surface of the carrier, in the region of the position where the patient rests. The slot 28 needs to be relatively wide, to enable the displacement of the L-shaped profile through the slit to enable its application. To fixate the position of the profile into the slot and to prevent displacement, it is necessary to apply a positioning block 29 or the like into the slot. The presence of the slot, the L-shaped profile and the positioning block disrupt the homogeneity of the material of the carrier at the position where the patient is located, so that there is a risk that the desired radiation dose into the tissue is not achieved.

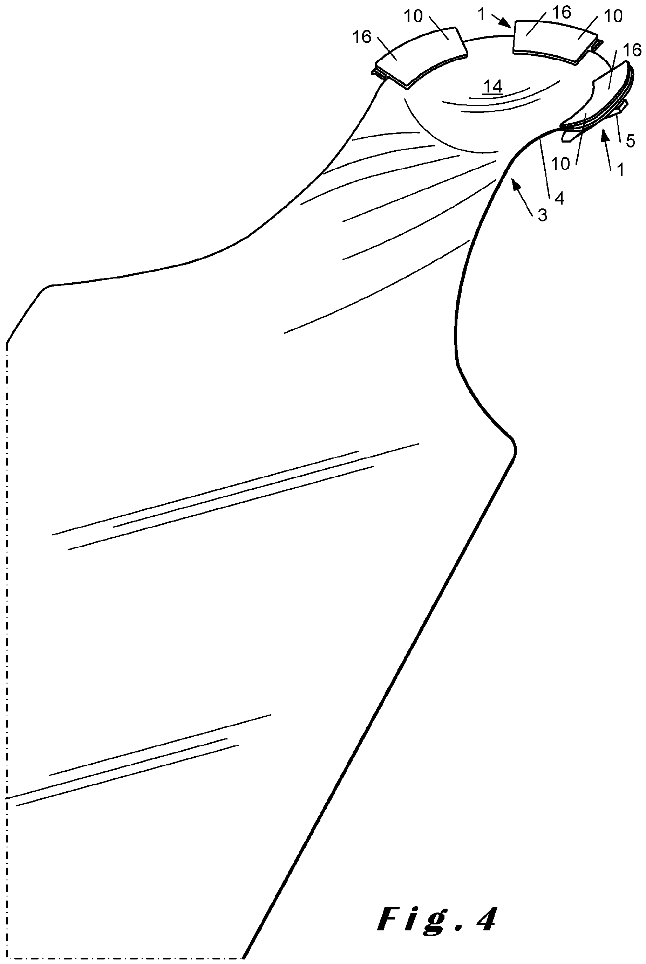

A view to a upper side of a preferred embodiment of a carrier or base plate 3 for supporting a patient in radiation therapy is shown in figure 4. The preferred embodiment shown in figure 4 of the carrier 3 contains a top support surface 14 for supporting the patient. With "along" is meant that the connection means are located at a distance of the edge 4 of the carrier 3 or may be provided in one piece with the carrier, dependent on the nature of the connection means 21 on the profile. Along the edge of the carrier 3, preferably connection means 13 extend at least at one position, that are provided to cooperate with the connection means 1 for the connection of the immobilisation mask 2 with the carrier 3. Along the edge 4 of the carrier 3, there is provided preferably on at least one position, an opening 13 for receiving connection means 1 for attaching the immobilisation mask 2 to the carrier 3. More preferable, two or more openings 13 are provided. The amount of openings 13 is usually chosen such that a sufficient amount of profiles 1 can be applied to enable a stable and accurate fixation of the position and posture of the body part of the patient and to allow not more than the desired range of motion. The openings 13 may be applied at random positions along the edge 4 of the carrier. However, preferably, the openings 13 are applied symmetrically relative to the longitudinal axis of the carrier to attain optimal distribution of the tension and tensile force over the immobilisation mask 2 and to enable optimal derivation thereof to the carrier 3 (see fig. 4). It is further possible to choose the amount of openings 3 such that the carrier 3 is suitable for immobilizing multiple body parts with varying size. Every other arrangement of openings 13 considered suitable by the person skilled in the art may be chosen.

Furthermore, the carrier 3 along the edge 4 is preferably, at least one position 20, deformed for receiving a profile 1 for attaching the immobilisation mask 2 to the carrier 3. The deformation 20 is preferably such that it is sufficiently large to receive the upper lip 10 of the profile 1 in such a way that the upper surface of the upper lip 10 is flush with the support surface 14 of the base plate or carrier 3 to increase the comfort of the patient. The presence of the deformation 20 hampers the displacement of the profile 1 along and relative to the edge of the carrier 4 and contributes in this way to the provision of a stable, accurate and reproducible connection to the immobilisation mask 2 with the carrier. The deformation also counteracts the possibility of rotation of the profile 1 relative to the carrier 3. Preferably, two or more deformations 20 are provided. The number of deformations 20 is usually chosen such that a sufficient amount of profiles can be applied to enable a stable and accurate fixation of the position and posture of the body part of the patient and to allow no more than the desired range of motion. The deformations 20 can be located at random positions along the edge of the carrier. However, preferably the deformations 20 are applied with some symmetry to realize optimal distribution of tension and tensile force over the immobilisation mask 2 and to enable optimal derivation thereof to the carrier 3 (see fig. 4).

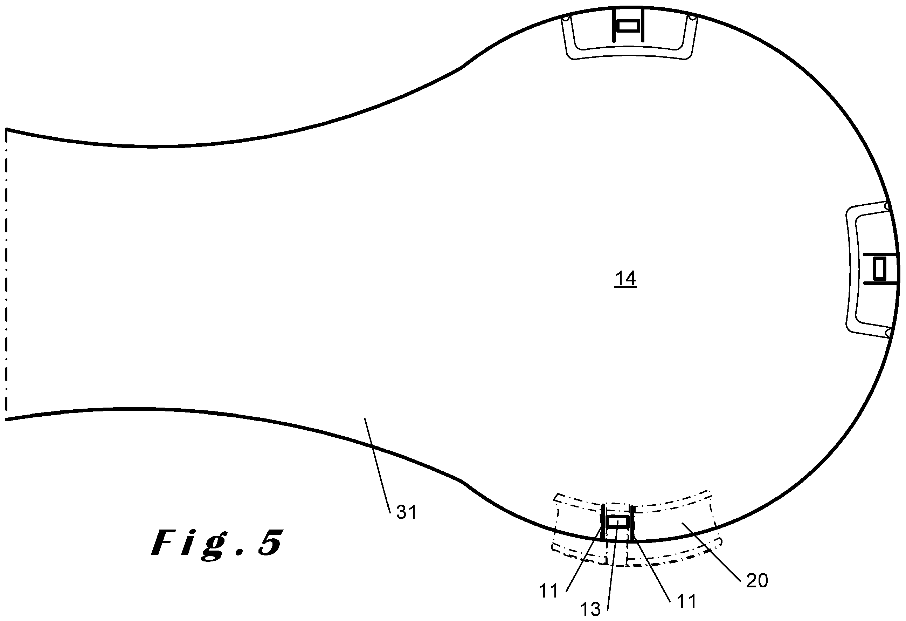

The dimensions and shape of the deformations 20 may be random. The deformation 20 preferably has the shape of a recess relative to the upper side of the carrier. In other words, the upper surface of the deformation 20 is preferably lowered relative to the upper surface 14 of the carrier. This is shown in figure 5. Preferably, the dimensions of the deformations 20 in the longitudinal direction along the edge 4 of the carrier and the dimensions of the deformation 20 in the traverse direction on the carrier 4 are aligned with the dimensions of the profile 1 or connection means that are provided to be incorporated into this deformation. The deformation 20 preferably has nearly the same dimensions as the profile 1 or is slightly larger than the profile 1, such that the profile 1 or at least the upper lip 10 of the profile can be incorporated in the deformation 20. The deformation 20 may function, at the application of the profile, as a guide for guiding the displacement of the profile 1 during the establishment of the connection between the mask 2 and the carrier 3. Since the raised edge of the deformation forms a barrier, the displacement of the profile 1, once installed, is opposed, and the deformation 20 contributes to the stable and accurate fixation of the position and posture of the body part of the patient so as to allow no more than the intended range of motion.

A view to the lower side of a preferred embodiment of a commonly used carrier 3 for supporting a patient, especially in proton therapy, is shown in figure 5. At a position between the edge 4 of the carrier and the opening 13, a guide 11 is preferably provided at the lower side of the carrier for guiding the displacement of the profile 1 at the establishment of the connection of the immobilisation mask 2 with the carrier 3, in particular for guiding the displacement of the first lower lip 5 on the profile 1. A preferred embodiment of the guide 11 has the shape of two opposite protrusions or rails, in between which the lip 5 of the profile 1 is displaceable at the establishment of the connection. Once the connection has been established, the protrusions counteract a displacement of the lip 5 in a direction along the edge and therefore contribute to a stable and accurate fixation of the position and posture of the body part of the patient. Instead of the protrusions or rails 11, an alternative guide, for example a slot or any other alternative deemed suitable by the person skilled in the art, may be used that is adapted to embodiment of the lower lip 5.

The connection means preferably comprise a profile 1 with first connection means 21 at a first side of the profile along which the profile is connectable to the carrier 3, and second connection means 22 at a second side of the profile 1 opposite the first side along which the profile 1 is connectable to the immobilisation mask 2. The first connection means 21 are provided, in the connected state, to engage an outer edge of the carrier. Such connection means enable to establish a connection between the immobilisation mask 2 and the carrier 3, also when the profile is subjected to high tension. The first connection means 21 preferably engage in the thickness direction the outer edge of the carrier. In a possible embodiment, the connection means 21 exercise a clamping force onto the carrier. In another possible embodiment, the first connection means 21 exercise a clamping force onto the carrier 3.

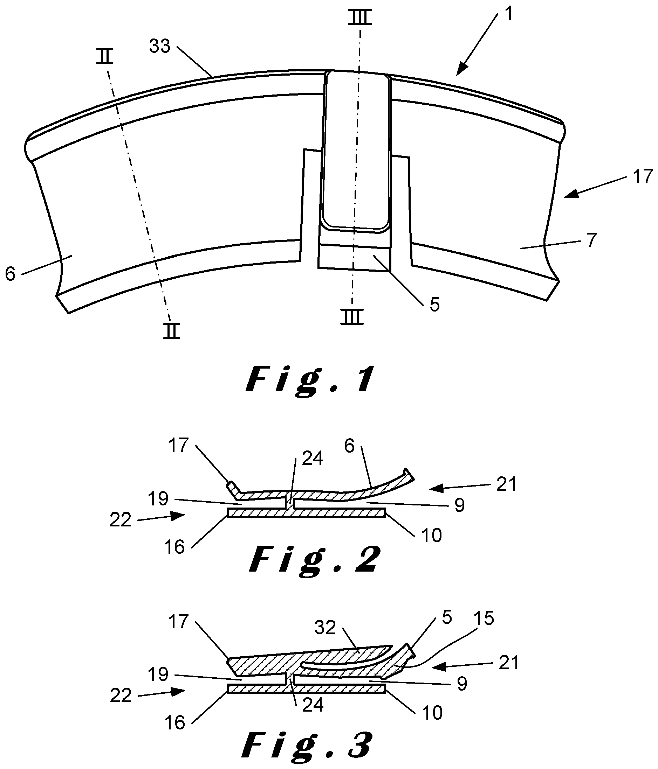

The first connection means 21 preferably comprise an upper lip 10 that is provided to extend, in the connected state, along an upper surface or side of the carrier 14. Preferably, the upper lip 10 is incorporated in a corresponding deformation 20 along the upper surface 14 of the carrier 3. To enable a greater transmission of forces between the immobilisation mask 2 and the carrier 3, the upper lip 10 preferably extends in the longitudinal direction along the edge 4 of the carrier 3. The upper lip 10 may, for example, be curved to optimally follow the shape of the immobilisation mask 2, the upper lip 10 may however also be flat and rectangular or may take any other appropriate shape. The edge 33 of the upper lip may, for example, be curved to optimally follow the shape of the edge of the immobilisation mask as shown in figure 1. The upper lip 10 increases the stability of the mask in the connected state with the carrier, since a tilting movement of the profile is counteracted.

The first connection means 21 preferably also comprise at least one lower lip 5 that is provided to extend in the connected state along a lower surface or lower side of the carrier 31. More preferably, the first connection means 21 comprise a lower lip 5 that is provided on the side directed to the carrier with a protrusion 15 that is provided, in the connected state, to engage corresponding connection means on the lower side of the carrier 3. Preferably, protrusion 15 engages a corresponding opening 13 in the carrier 3. The opening 13 may extend throughout the entire thickness of the carrier 3 or over a section thereof.

According to the invention, the first connection means 21 further comprise on a first side of the lower lip 5 a second lower lip 6 which preferably extends in the longitudinal direction along the edge of the carrier 3. The first connection means 21 further comprise at a second side of the lower lip 5 opposite the first side in transverse direction of the lower lip 5, a third lower lip 7 which preferably extends in the longitudinal direction along the edge of the carrier 3. With a resiliently arranged lip 5, an easily detachable connection is provided between the profile 1, in particular between the profile and the carrier 3. The second and third lip 6, 7 may have the same or different shapes, but preferably have a corresponding shape. The second and third lower lips 6, 7 may, for example, be curved to optimally follow the shape of the immobilisation mask, they may however also be rectangular or may take any other appropriate shape. The presence of the second and third lower lip 6, 7 increases the stability of the mask in the connected state with the carrier, since a tilting movement of the profile 1 is counteracted.

Preferably, the outer surface of lip 5 further contains a second lip 32 that counteracts or even prevents a movement of the lip 5 that is directed away from the space 9.

The opening 13 can be arranged centrally in the deformation 20, or asymmetrically.

The at least one upper lip 10 and three lower lips 5, 6, 7, of the first connection means 21 are preferably arranged at a distance from one another and are connected to each other at a connection position 24. Thereby, the first distance between the at least one upper lip 10 and lower lips 5, 6, 7, is preferably chosen such that, at one first side of the connection position 2, it is sufficiently large for receiving a section of the carrier 3 that extends along the edge 4 thereof. The second connection means 22 preferably contain also an upper and a lower lip 16, 17 that are connected to each other at the connection position 24. The upper and lower lip 16, 17 are preferably arranged at a second distance from each other that is sufficiently large for the inclusion of the material along the edge of the immobilisation mask. The first distance can be equal to the second distance, or may be different from it. Connection position 24 connects the first 21 and second connection means 22.

The first connection means 21 for connecting the profile to the carrier, preferably extends in a first direction relative to the connection position 24, in particular in the direction of the carrier 3. The second connection means 22 that connect the profile 1 to the immobilisation mask preferably extend in opposite direction relative to the first connection means 21 and the connection position 24, in particular in the direction of the immobilisation mask.

The carrier 3 may be provided in every material deemed suitable by the person skilled in the art. The carrier is for example conducted as a plate of carbon fibres impregnated with epoxy resin. However, other materials may be used.

The profile 1 may be provided in one piece with the immobilisation mask 2 or as a separate component. Preferably, it is provided in one piece with the mask 2 in order to absorb higher tensile forces on the mask with minimal risk of releasing the profile 1. The profile 1 may be provided in another material than the mask, such that for both components the material may be chosen that is optimally adapted to the selected function it performs. This offers, for example, the possibility to choose the material for the mask such that it may directly be shaped to the patient, and to choose the material of the profile 1 such that it exhibits an optimal mechanical strength and minimal deformation to ensure an accurate and reproducible positioning.

Suitable materials for manufacturing the immobilisation mask are for example thermoplastic elastomers, thermoplastic polyurethane, thermoplastic polyisoprene, thermoplastic polyesters, thermoplastic polyolefins, polyvinyl chloride, polystyrene, or a blend of two or more of these materials. Preferred are these polymers that have a relatively low softening temperature wherein the material is deformable, such that these are exceptionally suitable for the manufacturing of immobilisation mask that can directly be formed onto the body part to be immobilized. The body part to be immobilized then serves as a mould for shaping the immobilisation mask. Examples of suitable thermoplastic polyolefins comprise polyethylene, polypropylene or ethylene-propylene co-polymers. Examples of suitable thermoplastic polyesters comprise polyethylene vinyl acetate, polyacrylates or polymethacrylate, polymeric fatty acid esters, in particular poly-ε-caprolactone, for example poly-ε-caprolactone such as available at Perstorp (UK) under the trade name Capa®. Preferred are the polymers of the group of thermoplastic polyurethane, isotactic polypropylene, copolymers of ethylene with 1-butene, a copolymer of ethylene with 1-octene, poly-ε-caprolactone, poly-C-caprolactone containing thermoplastic polyurethane, as well as mixtures of two or more of these materials. Poly-ε-caprolactone is especially preferred because it has a low melting point and is deformable at temperatures that the human body can tolerate.

Suitable materials for manufacturing the profile are ABS, polyoxymethylene, polyamide or other equivalent materials.

An immobilisation mask for immobilizing a body part of the patient in a previously determined posture of the carrier 3 is provided with a profile 1 as described above, for connecting the mask 2 to the carrier 3 for supporting the patient. Preferably, the profile 1 is attached to the mask 2 along the edge of the mask 12, by incorporating the edge 12 of the mask in the second space 19 at the second connection means 22. Attachment can be done, for example, by gluing, by welding, by means of a mechanical connection or by any other connection deemed suitable by the person skilled in the art. Preferably, the connection is established by gluing or welding. Preferably, the profile 1 is as described above. To enable an optimal positioning and immobilizing along the edge of the mask, usually multiple profiles 1 will be applied.

A profile 1 for connecting an immobilisation mask 2 to the carrier 3 is provided, wherein the mask 2 is provided for immobilizing a body part of a patient in a predetermined posture on the carrier. Preferably, the profile 1 is as described above.

Connecting an immobilisation mask 2 to the carrier 3 upon which a patient rests, for immobilizing for example the head of the patient occurs by covering the head of the patient with the immobilisation mask that was shaped to the head of the patient. When use is made of an immobilisation mask on which along the edge a plurality of profiles are attached, a connection is established by incorporating the edge 4 of the carrier 3 in the first space 9 of the profile 1 at the side of the first connection means 21, preferably at the height of a recess 20 in the carrier 3. When establishing the connection, the profile is usually placed transversal to the edge 4 of the carrier 3 in the direction of the middle of the carrier 3. The displacement of the profile 1 may be guided by the edges of the recess or deformation 20 at the upper side of the carrier 14 and by the guide 11 at the lower side 31 of the carrier that guides the displacement of the first lip 5 along the lower side 31 of the carrier 3 till the protrusion 15 engages the carrier at the first lip 5 in the corresponding opening 13.

### LIST OF REFERENCE NUMBERS.

- 1.: profile
- 2.: immobilisation mask
- 3.: carrier for patient
- 4.: edge carrier for patient
- 5.: first lower lip
- 6.: second lower lip
- 7.: third lower lip
- 8.:
- 9.: first space
- 10.: upper lip
- 11.: guide
- 12.: edge mask
- 13.: opening in edge carrier for patient
- 14.: support surface carrier for patient
- 15.: protrusion on first lower lip
- 16.: upper lip second connection means
- 17.: lower lip second connection means
- 18.:
- 19.: second space
- 20.: deformation in carrier
- 21.: first connection means
- 22.: second connection means
- 23.:
- 24.: connection position
- 25.: L- shaped profile
- 26.: immobilisation mask
- 27.: carrier
- 28.: slot in carrier
- 29.: EVA block -
- 30.:
- 31.: lower side carrier
- 32.: second lip
- 33.: edge upper lip

## Claims

1. Assembly for radiation therapy, for example proton therapy, comprising:
a carrier (3) for supporting a patient,
an immobilisation mask (2) connectable with the carrier (3) for immobilizing a body part of the patient onto the carrier (3) and
connection means for connecting the carrier (3) with the immobilisation mask (2),
wherein the immobilisation mask is manufactured from thermoplastic material,
wherein the connection means contain at least one profile (1) that on a first side contains first connection means (21) along which the profile (1) is connectable to the carrier (3) and on a second side opposite the first, contains second connection means (22) along which the profile (1) is connectable to the immobilisation mask (2), wherein the first connection means (21) are provided, in the connected state, to engage corresponding connection means (13) along the outer edge (4) of the carrier (3)
**characterized in that** the first connection means (21) comprise a first lower lip (5) that is provided, in the connected state, to extend along the carrier (3),
further **characterized in that** the first connection means (21) comprise a second and third lower lip (6, 7) at opposite sides of the first lower lip (5), wherein the second and third lower lip (6, 7) are provided to extend along a lower side (31) of the carrier (3),
further **characterized in that** the first connection means (21) comprise an upper lip (10) that is provided, in the connected state, to extend along an upper surface (14) of the carrier (3), and the first lower lip (5) is provided, in the connected state, to extend along the lower side (31) of the carrier (3), wherein the edge of the carrier is held between said first lower lip (5) and said upper lip (10) and
further **characterized in that** the first lower lip (5) is arranged resiliently.

2. Assembly according to claim 1, wherein the immobilisation mask (2) is manufactured from a thermoplastic material, chosen from the group, consisting of thermoplastic elastomers, thermoplastic polyurethane, thermoplastic polyisoprene, thermoplastic polyesters, thermoplastic polyolefins, polyvinyl chloride, polystyrene, or a blend of two or more of these materials.

3. Assembly according to claim 2, wherein the immobilisation mask (2) is manufactured from thermoplastic polyesters.

4. Assembly according to claim 2 or 3, wherein the immobilisation mask (2) is manufactured from suitable thermoplastic polyolefins, selected from the group consisting of polyethylene, polypropylene, or ethylene-propylene co-polymers, selected from the group of polyethylene vinyl acetate, polyacrylates or polymethacrylate, polymeric fatty acid esters, in particular poly-ε-caprolactone, polymers found to be suitable from the group of thermoplastic polyurethane, isotactic polypropylene, copolymers of ethylene with 1-butene, a copolymer of ethylene with 1-octene, poly-ε-caprolactone, poly-ε-caprolactone containing thermoplastic polyurethane, as well as mixtures of two or more of these materials.

5. Assembly according to any one of the preceding claims, wherein the immobilisation mask (2) is manufactured from polymers that have a softening temperature at which the material is deformable, such that they are suitable for the manufacturing of immobilisation masks (2) that can be directly shaped onto the body part to be immobilized, wherein the body part to be immobilized serves as mould for shaping the immobilisation mask (2).

6. Assembly according to any one of the preceding claims, wherein the immobilisation mask (2) is manufactured from poly-ε-caprolactone.

7. Assembly according to any one of the preceding claims, wherein the first connection means (21) of the profile (1) and the outer edge (4) of the carrier (3) contain complementary, cooperating connection means (4, 15).

8. Assembly according to any one of the preceding claims, wherein the first connection means (21), in the connected state, engage the corresponding connection means (13) along the outer edge (4) of the carrier (3).

9. Assembly according to any one of the preceding claims, wherein the first lower lip (5) at a side directed to the carrier (3) is provided with a protrusion (15) that is provided, in the connected state, to engage the corresponding connection means (13) along the outer edge (4) of the carrier (3).

10. Assembly according to the preceding claim,wherein the corresponding connection means (13) along the outer edge (4) of the carrier (3) comprise an opening (13) for receiving the protrusion (15).

11. Assembly according to any one of the preceding claims, wherein at the lower side (31) of the carrier a guide (11) is provided for guiding the displacement of the first lower lip (5) to the position (13) where the first lower lip (5) of the carrier (3) engages at the establishment of the connection.

12. Assembly according to any one of the preceding claims, wherein the carrier (3) is deformed (20) along the edge (4) for receiving the lower lip (10).

13. Assembly according to any one of the preceding claims, wherein the lower lip (10) is elongated in a direction which extends along the edge (4) of the carrier.

14. Use of the assembly according to any one of the preceding claims, for immobilizing a body part of a patient on a carrier with radiation therapy, for example proton therapy.

## Patentansprüche

1. System für die Strahlentherapie, zum Beispiel Protonentherapie, welches Folgendes umfasst:
eine Patientenliege (3) zur Unterstützung eines Patienten,
eine Immobilisierungsmaske (2), verbindbar mit der Patientenliege (3), um einen Körperteil des Patienten auf der Patientenliege (3) zu immobilisieren, und
Verbindungsmittel, um die Patientenliege (3) mit der Immobilisierungsmaske (2) zu verbinden,
wobei die Immobilisierungsmaske aus thermoplastischem Material hergestellt ist,
wobei die Verbindungsmittel zumindest ein Profil (1) enthalten, das an einer ersten Seite ein erstes Verbindungsmittel (21) enthält, entlang dessen das Profil (1) mit der Patientenliege (3) verbindbar ist, und an einer zweiten Seite gegenüber der Ersten ein zweites Verbindungsmittel (22) enthält, entlang dessen das Profil (1) mit der Immobilisierungsmaske (2) verbindbar ist, wobei das erste Verbindungsmittel (21) bereitgestellt ist, um, im verbundenen Zustand, in das entsprechende Verbindungsmittel (13) entlang des äußeren Randes (4) der Patientenliege (3) einzugreifen,
**dadurch gekennzeichnet, dass**
das erste Verbindungsmittel (21) eine erste untere Lippe (5) umfasst, die, in verbundenem Zustand, bereitgestellt ist, um sich entlang der Patientenliege (3) zu erstrecken,
ferner **dadurch gekennzeichnet, dass** das erste Verbindungsmittel (21) eine zweite und eine dritte untere Lippe (6, 7) an gegenüberliegenden Seiten der ersten unteren Lippe (5) umfasst, wobei die zweite und die dritte untere Lippe (6, 7) bereitgestellt sind, um sich entlang einer unteren Seite (31) der Patientenliege (3) zu erstrecken,
ferner **dadurch gekennzeichnet, dass** das erste Verbindungsmittel (21) eine obere Lippe (10) umfasst, die, in verbundenem Zustand, bereitgestellt ist, um sich entlang einer oberen Oberfläche (14) der Patientenliege (3) zu erstrecken, und dadurch, dass die erste untere Lippe (5), in verbundenem Zustand, bereitgestellt ist, um sich entlang der unteren Seite (31) der Patientenliege (3) zu erstrecken, wobei der Rand der Patientenliege zwischen der erwähnten ersten unteren Lippe (5) und der erwähnten oberen Lippe (10) gehalten wird, und
ferner **dadurch gekennzeichnet, dass** die erste untere Lippe (5) federnd angeordnet ist.

2. System nach Anspruch 1, wobei die Immobilisierungsmaske (2) aus einem thermoplastischen Material hergestellt ist, ausgewählt aus der Gruppe bestehend aus thermoplastischen Elastomeren, thermoplastischem Polyurethan, thermoplastischem Polyisopren, thermoplastischen Polyestern, thermoplastischen Polyolefinen, Polyvinylchlorid, Polystyrol, oder einer Mischung von zwei oder mehr dieser Materialien.

3. System nach Anspruch 2, wobei die Immobilisierungsmaske (2) aus thermoplastischen Polyestern hergestellt ist.

4. System nach Anspruch 2 oder 3, wobei die Immobilisierungsmaske (2) aus geeigneten thermoplastischen Polyolefinen hergestellt ist, ausgewählt aus der Gruppe bestehend aus Polyethylen, Polypropylen, oder Ethylen-Propylen-Copolymeren, ausgewählt aus der Gruppe von Polyethylen-Vinylacetat, Polyacrylaten oder Polymethacrylat, polymerischen Fettsäureestern, insbesondere Poly-ε-Caprolacton, für geeignet befundenen Polymeren aus der Gruppe von thermoplastischem Polyurethan, isotaktischem Polypropylen, Copolymeren von Ethylen mit 1-Buten, einem Copolymer von Ethylen mit 1-Octen, Poly-ε-Caprolacton, Poly-ε-Caprolacton, das thermoplastisches Polyurethan enthält, sowie Mischungen von zwei oder mehr dieser Materialien.

5. System nach irgendeinem der vorigen Ansprüche, wobei die Immobilisierungsmaske (2) aus Polymeren hergestellt ist, die eine Erweichungstemperatur haben, bei der das Material verformbar ist, sodass sie für die Herstellung von Immobilisierungsmasken (2) geeignet sind, die direkt auf dem Körperteil geformt werden können, der immobilisiert werden soll, wobei der Körperteil, der immobilisiert werden soll, als Formstück zum Formen der Immobilisierungsmaske (2) dient.

6. System nach irgendeinem der vorigen Ansprüche, wobei die Immobilisierungsmaske (2) aus Poly-ε-Caprolacton hergestellt ist.

7. System nach irgendeinem der vorigen Ansprüche, wobei das erste Verbindungsmittel (21) des Profils (1) und der äußere Rand (4) der Patientenliege (3) komplementäre, damit zusammenwirkende Verbindungsmittel (4, 15) enthalten.

8. System nach irgendeinem der vorigen Ansprüche, wobei das erste Verbindungsmittel (21), im verbundenen Zustand, in das entsprechende Verbindungsmittel (13) entlang des äußeren Randes (4) der Patientenliege (3) eingreift.

9. System nach irgendeinem der vorigen Ansprüche, wobei die erste untere Lippe (5) an einer der Patientenliege (3) zugewandten Seite mit einem Überstand (15) versehen ist, der, in verbundenem Zustand, bereitgestellt ist, um in das entsprechende Verbindungsmittel (13) entlang des äußeren Randes (4) der Patientenliege (3) einzugreifen.

10. System nach dem vorigen Anspruch, wobei das entsprechende Verbindungsmittel (13) entlang des äußeren Randes (4) der Patientenliege (3) eine Öffnung (13) für die Aufnahme des Überstands (15) umfasst.

11. System nach irgendeinem der vorigen Ansprüche, wobei an der unteren Seite (31) der Patientenliege (3) eine Führung (11) bereitgestellt ist, um die Versetzung der ersten unteren Lippe (5) an die Position (13) zu führen, wo die erste untere Lippe (5) der Patientenliege (3) bei der Herstellung der Verbindung eingreift.

12. System nach irgendeinem der vorigen Ansprüche, wobei die Patientenliege (3) entlang des Randes (4) verformt ist (20), um die untere Lippe (10) aufzunehmen.

13. System nach irgendeinem der vorigen Ansprüche, wobei die untere Lippe (10) in eine Richtung verlängert ist, die sich entlang des Randes (4) der Patientenliege erstreckt.

14. Verwendung des Systems nach irgendeinem der vorigen Ansprüche, um einen Körperteil eines Patienten auf einer Patientenliege bei Strahlentherapie, zum Beispiel Protonentherapie, zu immobilisieren.

## Revendications

1. Ensemble de radiothérapie, par exemple une thérapie protonique, comprenant :
un support (3) pour le support d'un patient,
un masque d'immobilisation (2) pouvant être raccordé au support (3) pour l'immobilisation d'une partie de corps du patient sur le support (3) et des moyens de raccordement pour le raccordement du support (3) avec le masque d'immobilisation (2), dans lequel le masque d'immobilisation est fabriqué en matériau thermoplastique,
dans lequel les moyens de raccordement contiennent au moins un profil (1) qui sur un premier côté contient des premiers moyens de raccordement (21) le long desquels le profil (1) peut être raccordé au support (3) et sur un second côté opposé au premier, contient des seconds moyens de raccordement (22) le long desquels le profil (1) peut être raccordé au masque d'immobilisation (2), dans lequel les premiers moyens de raccordement (21) sont prévus, à l'état raccordé, pour mettre en prise des moyens de raccordement correspondants (13) le long de l'arête extérieure (4) du support (3),
**caractérisé en ce que** les premiers moyens de raccordement (21) comprennent une première lèvre inférieure (5) qui est prévue, à l'état raccordé, pour s'étendre le long du support (3), **caractérisé en outre en ce que** les premiers moyens de raccordement (21) comprennent une deuxième et une troisième lèvre inférieure (6, 7) sur des côtés opposés de la première lèvre inférieure (5), dans lequel les deuxième et troisième lèvres inférieures (6, 7) sont prévues pour s'étendre le long d'un côté inférieur (31) du support (3), **caractérisé en outre en ce que** les premiers moyens de raccordement (21) comprennent une lèvre supérieure (10) qui est prévue, à l'état raccordé, pour s'étendre le long d'une surface supérieure (14) du support (3), et la première lèvre inférieure (5) est prévue, à l'état raccordé, pour s'étendre le long du côté inférieur (31) du support (3), dans lequel l'arête du support est maintenue entre ladite première lèvre inférieure (5) et ladite lèvre supérieure (10) et
**caractérisé en outre en ce que** la première lèvre inférieure (5) est agencée de manière élastique.

2. Ensemble selon la revendication 1, dans lequel le masque d'immobilisation (2) est fabriqué en un matériau thermoplastique, choisi dans le groupe composé d'élastomères thermoplastiques, polyuréthane thermoplastique, polyisoprène thermoplastique, polyesters thermoplastiques, polyoléfines thermoplastiques, chlorure de polyvinyle, polystyrène ou un mélange de deux ou plus de ces matériaux.

3. Ensemble selon la revendication 2, dans lequel le masque d'immobilisation (2) est fabriqué en polyesters thermoplastiques.

4. Ensemble selon la revendication 2 ou 3, dans lequel le masque d'immobilisation (2) est fabriqué en polyoléfines thermoplastiques adaptés, sélectionnés dans le groupe composé de polyéthylène, polypropylène, ou copolymères d'éthylène-propylène, sélectionnés dans le groupe d'acétate de vinyle de polyéthylène, polyacrylates ou polyméthacrylate, esters d'acide gras polymères, en particulier poly-ε-caprolactone, des polymères qui conviennent dans le groupe de polyuréthane thermoplastique, polypropylène isotactique, copolymères d'éthylène avec 1-butène, un copolymère d'éthylène de 1-octène, poly-ε-caprolactone, poly-ε-caprolactone contenant du polyuréthane thermoplastique, ainsi que des mélanges de deux ou plus de ces matériaux.

5. Ensemble selon l'une quelconque des revendications précédentes, dans lequel le masque d'immobilisation (2) est fabriqué en polymères qui présentent une température de ramollissement à laquelle le matériau est déformable de sorte qu'ils soient adaptés à la fabrication de masques d'immobilisation (2) qui peuvent être directement formés sur la partie de corps à immobiliser, dans lequel la partie de corps à immobiliser sert de moule pour la formation du masque d'immobilisation (2).

6. Ensemble selon l'une quelconque des revendications précédentes, dans lequel le masque d'immobilisation (2) est fabriqué en poly-ε-caprolactone.

7. Ensemble selon l'une quelconque des revendications précédentes, dans lequel les premiers moyens de raccordement (21) du profil (1) et l'arête extérieure (4) du support (3) contiennent des moyens de raccordement coopérants, complémentaires (4, 15).

8. Ensemble selon l'une quelconque des revendications précédentes, dans lequel les premiers moyens de raccordement (21), à l'état raccordé, mettent en prise les moyens de raccordement correspondants (13) le long de l'arête extérieure (4) du support (3).

9. Ensemble selon l'une quelconque des revendications précédentes, dans lequel la première lèvre inférieure (5) sur un côté dirigé vers le support (3) est dotée d'une saillie (15) qui est prévue, à l'état raccordé, pour mettre en prise les moyens de raccordement correspondants (13) le long de l'arête extérieure (4) du support (3).

10. Ensemble selon la revendication précédente, dans lequel les moyens de raccordement correspondants (13) le long de l'arête extérieure (4) du support (3) comprennent une ouverture (13) pour la réception de la saillie (15).

11. Ensemble selon l'une quelconque des revendications précédentes, dans lequel sur le côté inférieur (31) du support un guidage (11) est prévu pour le guidage du déplacement de la première lèvre inférieure (5) à la position (13) où la première lèvre inférieure (5) du support (3) vient en prise lors de l'établissement du raccordement.

12. Ensemble selon l'une quelconque des revendications précédentes, dans lequel le support (3) est déformé (20) le long de l'arête (4) pour la réception de la lèvre inférieure (10).

13. Ensemble selon l'une quelconque des revendications précédentes, dans lequel la lèvre inférieure (10) est allongée dans une direction qui s'étend le long de l'arête (4) du support.

14. Utilisation de l'ensemble selon l'une quelconque des revendications précédentes, pour l'immobilisation d'une partie de corps d'un patient sur un support avec la radiothérapie, par exemple la thérapie protonique.
